Europäisches Patentamt

European Patent Office

Office européen des brevets

(11) Publication number: **0 193 358**
**B1**

(12)

# EUROPEAN PATENT SPECIFICATION

(45) Date of publication of patent specification: **30.08.89**

(51) Int. Cl.⁴: **C 07 C 79/355, C 07 C 76/02**

(21) Application number: **86301234.0**

(22) Date of filing: **21.02.86**

(54) Method of preparing 3,3'-dinitrodiphenyl ether.

(30) Priority: **22.02.85 JP 32569/85**
**26.02.85 JP 35228/85**
**27.02.85 JP 36535/85**

(43) Date of publication of application:
**03.09.86 Bulletin 86/36**

(45) Publication of the grant of the patent:
**30.08.89 Bulletin 89/35**

(84) Designated Contracting States:
**CH DE FR GB IT LI NL**

(56) References cited:
**US-A-4 179 461**

**JOURNAL OF ORGANIC CHEMISTRY, vol. 41,
no. 9, 30th April 1976, pages 1560-1564; N.
KORNBLUM et al.: "Displacement of the nitro
group of substituted nitrobenzenes - a
synthetically useful process"**

(73) Proprietor: **MITSUI TOATSU CHEMICALS INC.**
**No. 2-5, Kasumigaseki 3-chome**
**Chiyoda-Ku Tokyo 100 (JP)**

(72) Inventor: **Yamaguchi, Keizaburo**
**600-1, Kamisakunobe Takatsu-ku**
**Kawasaki-shi Kanagawa-ken (JP)**
Inventor: **Yoshikawa, Yukihiro**
**4-10-8, Hisagi**
**Zushi-shi Kanagawa-ken (JP)**
Inventor: **Tamai, Shoji**
**2070, Iijima-cho Totsuka-ku**
**Yokohama-shi Kanagawa-ken (JP)**
Inventor: **Sugimoto, Kenichi**
**2882, Iijima-cho Totsuka-ku**
**Yokohama-shi Kanagawa-ken (JP)**
Inventor: **Tanabe, Yoshimitsu**
**2070, Iijima-cho Totsuka-ku**
**Yokohama-shi Kanagawa-ken (JP)**
Inventor: **Yamaguchi, Akihiro**
**1-13-24, Zaimokuza**
**Kamakura-shi Kanagawa-ken (JP)**

(74) Representative: **Hayward, Denis Edward Peter
et al**
**Lloyd Wise, Tregear & Co. Norman House
105-109 Strand
London WC2R 0AE (GB)**

# EP 0 193 358 B1

(56) References cited:
**BULLETIN DE LA SOCIETE CHIMIQUE DE
FRANCE, Table des Matières, 1952, pages
546-549; M. JULIA: "No. 128 - Synthèse de
xanthones disubstituées en -3 et -6"**

**PATENTS ABSTRACTS OF JAPAN, vol. 4, no.
106 (C-20) 588r, 30th July 1980, page 153 C 20;
& JP - A - 55 69 542 (MITSUI TOATSU KAGAKU
K.K.) 26-05-1980**

## Description

The present invention relates to a method for preparing 3,3'-dinitrodiphenylether which comprises a condensation reaction of m-dinitrobenzene in a dipolar aprotic solvent.

More particularly, this invention relates to a method for preparing 3,3'-dinitrodiphenylether by the condensation reaction of m-dinitrobenzene with m-nitrophenol in the dipolar aprotic solvent in the presence of a base, or by the self-condensation reaction of m-dinitrobenzene in the dipolar aprotic solvent in the presence of a catalytic amount of nitrite or a catalytic amount of m-nitrophenol and the base.

3,3'-Dinitrodiphenylether is a raw material for agricultural chemicals and medicines as well as an intermediate for 3,3'-diaminodiphenylether which is a raw material of polyamide, polyamide imide and polyimide.

3,3'-Dinitrodiphenylether is so far known to be prepared by the Ullmann's reaction of m-nitrophenol and m-bromonitrobenzene (Tomita et al., J. of the Pharmaceutical Soc., Japan. *75*, 1080 (1955); Ikawa et al., ibid *79*, 273 (1959); J. P. Critchley et al., J. Polymer Sci., Part A-1, *10*, 1793 (1972)).

These methods, however, have drawbacks such as low yields or accompanying danger of explosion. In addition, the largest disadvantage is the high price and the not ready availability of raw materials for industrial application.

The object of this invention is to provide a cheap and industrially applicable method of preparing 3,3'-dinitrodiphenylether.

An intense research effort was put out by the inventors of this invention to achieve this object. Unexpectedly, a new fact was found that 3,3'-dinitrodiphenylether could be derived from cheaply producible m-dinitrobenzene as the raw material.

Benzene derivatives having a pair of meta-located electron-withdrawing groups are generally known to have a low activity for nucleophilic displacement reactions. As to the reactions of dinitrobenzene, examples are known only of their reaction with the following alcohols. These examples are:

(1) m-Nitroanisole was obtained by reacting m-dinitrobenzene with sodium methoxide in hexamethylphosphoramide (N. Kornblum et al., J. Org. Chem., *41*, 1560—64 (1976)).

(2) m-Nitroanisole was obtained by reacting m-dinitrobenzene with sodium methoxide in chlorobenzene in the presence of phase-transfer catalysts (F. Montanari et al., Chem. & Ind. *19*, 412 (1982)).

(3) m-Nitrophenyl alkyl ethers were obtained by reacting m-dinitrobenzene with alcohols in diplar aprotic solvents in the presence of a carbonate or hydrogen carbonate of an alkali metal or trialkali metal phosphate (Kuroda et al., Tokkaisho 58-180461, 59-25353).

In the present invention m-dinitrobenzene was studied as to its use as a raw material for reacting with phenols when it had, in the past, been assumed unreactive and had rarely been examined. On reacting with m-nitrophenol, it has been found that a condensation of alkali metal m-nitrophenolate with m-dinitrobenzene proceeds in a dipolar aprotic solvent in the presence of a base and the generated nitrite leads in turn to a self-condensation reaction of m-dinitrobenzene. It has also been found in accordance with this reaction that 3,3'-dinitrodiphenylether can be prepared by a self-condensation reaction of m-dinitrobenzene in a dipolar aprotic solvent in the presence of a catalytic amount of nitrite or a catalytic amount of m-nitrophenol and a base.

It is quite unknown that m-dinitrobenzene can perform this self-condensation reaction in the presence of nitrite.

Examples are known of the self-condensation method in the case of aromatic nitro compounds. These examples are:

(1) 4,4'-Oxybis(N-methylphthalimide) is prepared from 4-nitro-N-methylphthalimide (R. J. Markezich et al., J. Org. Chem., *42*, 3431—34 (1977).

(2) 4,4'-Dicyanodiphenylether is prepared from 4-nitrobenzonitrile (E. Heinz et al., Ger. Offen., 2,037,781).

These examples, however, are related to those compounds having electron-withdrawing groups at the p-position. As to those compounds substituted at the m-position as in the method of this invention, examples are not known.

Accordingly, methods are quite unknown which can advantageously prepare diphenylether derivatives by a self-condensation reaction of m-dinitrobenzene or a condensation reaction of m-dinitrobenzene with phenols.

Also known are:—

(1) A method for preparing 4,4'-dinitrodiphenyl ether by reacting p-chloronitrobenzene with sodium nitrite in N-methylpyrrolidone and preparing 4,4'-dicyanodiphenylether from p-nitrobenzonitrile and p-chlorobenzonitrile under the same conditions (Ger. Offen., 2,037,781).

(2) A method for preparing 2,2'-dinitrodiphenylether by reacting o-nitrofluorobenzene with potassium hydrogen carbonate in N,N-dimethylacetamide in the presence of a phase-transfer catalyst (J. Org. Chem., *49*, 1125 (1984)).

(3) A method for preparing 4,4'-oxybis(N-methylphthalimide) by reacting 4-nitro-N-methylphthalimide or 4-fluoro-N-methylphthalimide with potassium nitrite in a dipolar aprotic solvent (J. Org. Chem., *42*, 3431 (1977).

The present invention provides a method for preparing 3,3'-dinitrodiphenylether by a condensation reaction of m-dinitrobenzene in a dipolar aprotic solvent in the presence of nitrite or in the presence of m-nitrophenol and a base.

In the reaction by the method of this invention, m-dinitrobenzene is considered to undergo a condensation reaction with the m-nitrophenol anion which is intermediately formed in the reaction of m-dinitrobenzene with nitrite or from m-nitrophenol and the base.

According to the method of this invention, a valuable compound, 3,3'-dinitrodiphenylether, can be prepared from the very cheap raw materials, m-dinitrobenzene and nitrite or m-nitrophenol.

Nitrite is sufficient in catalytic amounts. m-Nitrophenol may be used in catalytic amounts to promote self-condensation of m-dinitrobenzene with the nitrite formed, or in about an equivalent quantity with m-dinitrobenzene to produce the condensation reaction.

The method of this invention is notable in regard to the conversion ratio to and selectivity of the desired product, which is easy to purify, and capable of providing a high purity product in good yields. Therefore, the method of this invention is excellent in the industrial production of 3,3'-dinitrodiphenylether.

m-Dinitrobenzene, the raw material for use in the present invention, can be commercially and inexpensively prepared by the dinitration of benzene.

In one embodiment of the method of this invention, preferably nitrite is used for the condensation reaction of m-dinitrobenzene in the presence of a catalytic amount of a nitrite, which can be potassium nitrite, sodium nitrite, calcium nitrite, magnesium nitrite, lithium nitrite, barium nitrite, and ammonium nitrite. Nitrites of other metals can also be employed.

The quantity in use of the nitrite is 0.01—5 times by mol, preferably 0.1—2 times by mol of m-dinitrobenzene.

In another embodiment of the method of this invention, m-nitrophenol may be used, from a catalytic amount to more than an equivalent quantity, in order to carry out the condensation reaction of m-nitrophenol with a base. The preferred quantity of m-nitrophenol employed is in the range of 0.05—1.2 times by mol of m-dinitrobenzene, and a half mol or less is enough when a catalytic amount is used.

The base simultaneously used in the latter embodiment can be an alkali metal carbonate, hydrogen carbonate, hydroxide and alkoxide, including, for example, potassium carbonate, sodium carbonate, potassium hydrogen carbonate, potassium hydroxide, sodium hydroxide, lithium hydroxide, sodium methoxide, and potassium isopropoxide.

The quantity of the base used can satisfactorily be one equivalent or more of m-nitrophenol, normally in the range of 1—10 equivalents, and preferably 1—2 equivalents.

The solvents used for the method of this invention are dipolar aprotic solvents, which include for example, 1,3-dimethyl-2-imidazolidinone, hexamethylphosphoramide, N,N-dimethylacetamide, N,N-dimethylformamide, N-methylpyrrolidone, dimethylsulfoxide and sulfolane. Preferably used are 1,3-dimethyl-2-imidazolidinone, hexamethylphosphoramide and N,N-dimethylformamide. The quantity of these solvents employed is not critical, and normally is 1—15 times, preferably 3—10 times by weight of the raw materials.

Also in the method of this invention, catalysts which may be used to accelerate the reaction include copper compounds and phase-transfer catalysts such as crown ether, polyethylene glycol, quaternary ammonium salts or quaternary phosphonium salts.

In the method of this invention, reaction temperatures are normally in the range of 100—240°C, and preferably in the range of 130—200°C.

In the normal embodiment of the method of this invention, the self-condensation reaction by use of nitrite is carried out at the specified temperatures after raising the temperatures of all materials receiving the nitrite. The condensation reaction by use of m-nitrophenol and a base is conducted by charging the specified quantity of m-nitrophenol, the base and the solvents to form an alkali metal salt of m-nitrophenol before adding m-dinitrobenzene. This reaction may also be performed by simultaneously charging all the materials which have previously received the m-dinitrobenzene and raising the temperature. The method is of course not limited to these procedures and other embodiments may suitably be employed.

Regarding the procedures for removing water generated in the reaction system, there is a process for gradually discharging water out of the system during reaction by introducing a stream of nitrogen gas. But generally, very often employed is the process for azeotropically removing water from the reaction system with a small quantity of solvents such as, for example, benzene, toluene, xylene and chlorobenzene.

The end-point of the reaction can be determined by observing the decrease of raw materials with thin-layer chromatography, high performance chromatography or gas chromatography.

After ending the reaction, the resulting mixture is concentrated, or left as it is, and poured into water to separate crude 3,3'-dinitrodiphenylether. The crude product can easily be purified by recrystallizing from the solvents.

Examples

The invention will be illustrated further with respect to the following Examples.

Example 1

A reaction vessel with a stirrer, thermometer, and reflux condenser was charged with 84 grams (0.5 mol) of m-dinitrobenzene, 55.2 grams (0.8 mol) of sodium nitrite 450 ml of 1,3-dimethyl-2-imidazolidinone. The mixture was heated with stirring in a nitrogen atmosphere. The temperature was raised to 170—180°C and then kept constant for 18 hours to complete the reaction. The resultant reaction mixture was then poured in 2 l of water to separate brown solids. The precipitate was filtered, dried and recrystallized from benzene to obtain 36.6 grams of 3,3'-dinitrodiphenylether (56.3% yield). Further recrystallization from cyclohexane gave pale-brown prisms of pure product having a melting point of 126—128°C.

Results of elementary analysis were as follows.

Elementary analysis ($C_{12}H_8O_5N_2$)

|  | C | H | N |
|---|---|---|---|
| Calculated (%) | 55.39 | 3.10 | 10.77 |
| Found (%) | 55.11 | 2.96 | 10.62 |

Example 2

The same reaction vessel as Example 1 was equipped with a water separator and charged with 84 grams (0.5 mol) of m-dinitrobenzene, 30 grams (0.2 mol) of calcium nitrite monohydrate, 50 ml of benzene and 300 ml of hexamethylphosphoramide. The temperature of the mixture was raised with stirring in a nitrogen atmosphere. Azeotropically distilled water in the refluxing conditions of benzene was removed from the reaction system by the water separator. After completing the azeotropic dehydration, the temperature of the resultant mixture was gradually raised to 175—180°C while distilling off benzene, and kept constant for 8 hours to complete the reaction. After ending the reaction, the reaction mixture was filtered to remove inorganic salts and the like, followed by concentrating in a vacuum to recover solvents. The residual tarry material was poured into 500 ml of water and kept under continuous stirring to change it into small agglomerates of brown crystals. The agglomerates were filtered, dried and recrystallized from benzene to obtain 40.5 grams (62.3% yield) of 3,3'-dinitrodiphenylether.

Example 3

The procedure of Example 1 was repeated except 9 grams of potassium nitrite and 0.9 gram of 18-crown-6-ether catalyst were used in place of sodium nitrite. 37.5 Grams of 3,3'-dinitrodiphenylether were obtained (57.7% yield).

Example 4

The procedure of Example 1 was repeated except 63.7 grams of barium nitrite were used in place of sodium nitrite to obtain 36.7 grams (61% yield) of 3,3'-dinitrodiphenylether.

Example 5

A reaction vessel with a stirrer, thermometer, reflux condenser and water separator was charged with 7.0 grams (0.05 mol) of m-nitrophenol, 2.92 grams (0.05 mol) of 96% potassium hydroxide, 30 ml of benzene and 500 ml of 1,3-dimethyl-2-imidazolidinone. The mixture was warmed with stirring to remove azeotropically distilled water from the reaction system under the refluxing conditions of benzene. After completing the azeotropic dehydration, the temperature was reduced to 60°C and 84 grams (0.5 mol) of m-dinitrobenzene were added. The temperature was then raised in a nitrogen atmosphere and the reaction was carried out at 170—180°C for 18 hours.

After ending the reaction, the resultant mixture was concentrated in a vacuum to recover solvents. The residual tarry material was poured into 500 ml of water and kept under continuous stirring to change it into small agglomerates of brown crystals. The agglomerates were filtered, dried and recrystallized from benzene to obtain 39.2 grams (54.8% yield) of 3,3'-dinitrodiphenylether. Pale brown prisms of pure product were obtained by recrystallizing from ethylacetate. The prisms had a melting point of 127—128°C and results of elementary analysis were as follows.

Elementary analysis ($C_{12}H_8O_5N_2$)

|  | C | H | N |
|---|---|---|---|
| Calculated (%) | 55.39 | 3.10 | 10.77 |
| Found (%) | 55.12 | 3.31 | 10.71 |

Example 6

A reaction vessel with a stirrer and thermometer was charged with 20.9 grams (0.15 mol) of m-nitrophenol, 84 grams (0.5 mol) of m-dinitrobenzene, 13.8 grams (0.1 mol) of anhydrous potassium carbonate, 0.3 gram of copper powder and 350 ml of hexamethylphosphoramide. The mixture was heated with stirring in a nitrogen atmosphere and reacted at 180—190°C for 8 hours to complete the reaction.

After ending the reaction, the resultant reaction mixture was treated as Example 5 and 57.6 grams (63.6% yield) of 3,3'-dinitrodiphenylether were obtained.

Example 7

The procedure of Example 5 was repeated except 2.7 grams (0.05 mol) of sodium methoxide were used in place of potassium hydroxide and generated methanol was azeotropically distilled off with benzene. 39.7 Grams (55.5% yield) of 3,3'-dinitrodiphenylether were obtained.

Example 8

The procedure of Example 6 was repeated except 10.6 grams (0.1 mol) of sodium carbonate were used in place of anhydrous potassium carbonate, and 50.3 grams (59.5% yield) of 3,3'-dinitrodiphenylether were obtained.

Example 9

The same vessel as Example 5 was charged with 20.9 grams (0.15 mol) of m-nitrophenol, 6 grams (0.15 mol) of sodium hydroxide, 30 ml of benzene and 500 ml of N,N-dimethylformamide. The mixture was warmed with stirring to remove azeotropically distilled water from the reaction system under the refluxing conditions of benzene. After completing the azeotropic dehydration, the temperature was reduced to 60°C and 84 grams (0.5 mol) of m-dinitrobenzene were added. The same procedure was thereafter repeated as Example 5 and 34.8 grams (41.2% yield) of 3,3′-dinitrodiphenylether were obtained.

Example 10

A 2 l separable flask with a stirrer, thermometer, reflux condenser and water separator was charged with 139.1 grams (1 mol) of m-nitrophenol, 58.4 grams (1 mol) of 96% potassium hydroxide, 100 ml of benzene and 1,000 ml of N,N-dimethylformamide. The mixture was warmed with stirring to remove azeotropically distilled water from the reaction system under the refluxing conditions of benzene. After completing the azeotropic dehydration, the temperature was reduced to 60°C, and 168.1 grams (1 mol) of m-dinitrobenzene and 3.2 grams of copper powder were added. The temperature in the flask was then raised in a nitrogen atmosphere and the reaction was carried out for 10 hours at 150—153°C.

After ending the reaction, the resultant mixture was filtered to remove inorganic salts and the like and concentrated in a vacuum to recover solvents. The residual tarry material was poured into 2,000 ml of water and kept under continuous stirring to obtain small agglomerates of brown crystals. The agglomerates were filtered, dried and recrystallized from benzene to obtain 183.5 grams (70.6% yield) of 3,3′-dinitrodiphenylether. The purity of the product was 97% based on gas chromatography. The product was further recrystallized from a solvent mixture of benzene and alcohol, and purified product was obtained as pale brown prisms having a melting point of 127—128°C. Results of elementary analysis were as follows.

Elementary analysis ($C_{12}H_8O_5N_2$)

|  | C | H | H |
|---|---|---|---|
| Calculated (%) | 55.39 | 3.10 | 10.77 |
| Found (%) | 55.6 | 3.02 | 10.44 |

Example 11

A 2 l separable flask with a stirrer, thermometer, reflux condenser and water separator was charged with 146 grams (1.05 mol) of m-nitrophenol, 83 grams (0.6 mol) of anhydrous potassium carbonate, 168.1 grams (1.0 mol) of m-dinitrobenzene and 1,500 ml of 1,3-dimethyl-2-imidazolidinone. The reaction was carried out at 170—175°C for 5 hours.

After ending the reaction, the resultant mixture was poured into 5 l of water. Crude 3,3′-dinitrodiphenylether was obtained by filtering the separated small brown agglomerates.

The yield was 231 grams and the purity was 89% based on gas chromatography.

Example 12

The procedure of Example 10 was repeated except hexamethylphosphoramide was used in place of N,N-dimethylformamide, and gave 210.9 grams (81.1% yield) of 3,3′-dinitrodiphenylether having a purity of 98.2%.

Example 13

The same equipment as Example 10 was charged with 139.1 grams (1 mol) of m-nitrophenol, 54 grams (1 mol) of sodium methoxide and 1,000 ml of N,N-dimethylacetamide. The mixture was heated under a stream of nitrogen gas and generated methanol was distilled off.

The resultant reaction mixture was then cooled, and 168.1 grams (1 mol) of m-dinitrobenzene and 3.2 grams of copper powder were added, followed by reacting at 155—160°C for 14 hours.

After ending the reaction, the aftertreatment of Example 10 was repeated to obtain 146 grams (56.1% yield) of 3,3′-dinitrodiphenylether.

Example 14

The same procedure as Example 10 was repeated except 40 grams (1 mol) of sodium hydroxide and 1,200 ml of N-methylpyrrolidone were used in place of potassium hydroxide and N,N-dimethylformamide respectively and the reaction was carried out at 160—170°C for 18 hours. 131.5 Grams (50.5% yield) of 3,3′-dinitrodiphenylether were obtained.

Claims

1. A method for preparing 3,3′-dinitrodiphenylether which comprises conducting a condensation reaction of m-dinitrobenzene in a dipolar aprotic solvent.

2. The method of claim 1 wherein m-dinitrobenzene undergoes a self-condensation reaction in said dipolar aprotic solvent in the presence of a catalytic amount of nitrite.

3. The method of claim 1 wherein m-dinitrobenzene undergoes a self-condensation reaction in said dipolar aprotic solvent in the presence of a catalytic amount of m-nitrophenol and a base.

4. The method of claim 1 wherein m-dinitrobenzene undergoes said condensation reaction in said dipolar aprotic solvent in the presence of m-nitrophenol and a base.

5. The method of claim 1, claim 2, claim 3 or claim 4 wherein said dipolar aprotic solvent is N,N-dimethylformamide, 1,3-dimethyl-2-imidazolidinone or hexamethylphosphoramide.

6. The method of claim 1, claim 2, claim 3 or claim 4 wherein the temperature of said reaction is 130—200°C.

7. The method of claim 2 wherein said catalytic amount of nitrite is 0.1—2 times by mol of m-dinitrobenzene.

Patentansprüche

1. Verfahren zur Herstellung von 3,3′-Dinitrodiphenylether gekennzeichnet durch eine Kondensationsreaktion von m-Dinitrobenzol in einem dipolaren aprotischen Lösungsmittel.

2. Verfahren nach Anspruch 1, wobei m-Dinitrobenzol einer Selbstkondensationsreaktion in dem dipolaren aprotischen Lösungsmittel in Gegenwart einer katalytischen Menge an Nitrit unterliegt.

3. Verfahren nach Anspruch 1, wobei m-Dinitrobenzol einer Selbstkondensationsreaktion in dem dipolaren aprotischen Lösungsmittel in Gegenwart einer katalytischen Menge an m-Nitrophenol und einer Base unterliegt.

4. Verfahren nach Anspruch 1, wobei m-Dinitrobenzol der Kondensationsreaktion in der dipolaren aprotischen Lösung in Gegenwart von m-Nitrophenol und einer Base unterliegt.

5. Verfahren nach Anspruch 1, 2, 3 oder 4, wobei das dipolare aprotische Lösungsmittel N,N-Dimethylformamid, 1,3-Dimethyl-2-imidazolidinon oder Hexamethylphosphoramid ist.

6. Verfahren nach Anspruch 1, 2, 3 oder 4, wobei die Temperatur der Reaktion 130—200°C beträgt.

7. Verfahren nach Anspruch 2, wobei die katalytische Nitritmenge das 0,1—2 fache pro mol m-Dinitrobenzol ist.

**Revendications**

1. Un procédé pour préparer du 3,3'-dinitrodiphényléther qui comprend l'aspect de réaliser une réaction de condensation de m-dinitrobenzène dans un solvant dipolaire aprotique.

2. Le procédé de la revendication 1 dans lequel du m-dinitrobenzène subit une réaction d'autocondensation dans ledit solvant dipolaire aprotique en la présence d'une quantité catalytique de nitrite.

3. Le procédé de la revendication 1 dans lequel du m-dinitrobenzène subit une réaction d'autocondensation dans ledit solvant dipolaire aprotique en la présence d'une quantité catalytique de m-nitrophénol et d'une base.

4. Le procédé de la revendication 1 dans lequel du m-dinitrobenzène subit une réaction de condensation dans ledit solvant dipolaire aprotique en la présence de m-nitrophénol et d'une base.

5. Le procédé de la revendication 1, de la revendication 2, de la revendication 3 ou de la revendication 4 dans lequel ledit solvant dipolaire aprotique est du N,N-diméthylformamide, du 1,3-diméthyl-3-imidazolidinone ou de l'hexaméthylphosphoramide.

6. Le procédé de la revendication 1, de al revendication 2, de la revendication 3 ou de la revendication 4 dans lequel la température de ladite réaction est de 130—200°C.

7. Le procédé de la revendication 2 dans lequel ladite quantité catalytique de nitrite est de 0,1 à 2 fois par mole de m-dinitrobenzène.